# EUROPEAN PATENT SPECIFICATION

(11) **EP 2 516 916 B1**
(45) Date of publication and mention of the grant of the patent: **04.11.2015**
(21) Application number: 10803324.2
(22) Date of filing: 22.12.2010
(51) Int. Cl.: F16L 39/00, F28F 9/02

(54) **MALE BAYONET CONNECTOR**
BAYONETTSTECKER
CONNECTEUR À BAÏONNETTE MÂLE

(30) Priority: 23.12.2009 US 289545 P
(43) Date of publication of application: 31.10.2012
(73) Proprietor: Nordson Corporation, Westlake, OH 44145 (US)
(72) Inventor: LEWIS, Peter, D., Fort Collins Colorado 80525 (US); NARAYANAN, Ravikumar, Fort Collins Colorado 80525 (US); CAIRNS, Richards, W., Longmont Colorado 80504 (US); PHIPPS, Riley, M., Fort Collins Colorado 80521 (US)
(74) Representative: Eisenführ Speiser
(86) International application number: PCT/US2010/061896
(87) International publication number: WO 2011/079226

(56) References cited:
- WO-A2-2006/135666
- DE-C1- 4 138 064
- DE-U- 1 868 896
- US-A1- 2005 046 184
- US-A1- 2005 057 042

## Description

### BACKGROUND

### Technical Field

The present disclosure relates generally to the field of medical devices used for the transport of both gaseous and liquid fluids and more specifically, to a male bayonet connector for creating a releasable air and fluid seal connection between one or more sections of tubing and a female latch connector.

### Description of Related Art

Tubing sections, for example, medical tubing, must often be joined together to provide for gas and/or liquid fluid flow from one component to another. It is further often desirable to connect and disconnect tubing sections from one another. For example, when a patient's blood pressure is taken with an automatic blood pressure monitor, tubing from the blood pressure cuff (which is generally wrapped around the patient's arm) is connected to the tubing that is connected to the blood pressure monitor. To disconnect the cuff from the blood pressure monitor, it is desirable to merely detach the tubing section connected to the cuff from the tubing connected to the blood pressure monitor. Similarly, when providing intravenous fluids, it is often required to replace an empty fluid bag with a full fluid bag without removing the intravenous needle or stent from the patient. In order to switch between the first fluid bag and the second fluid bag, it is desirable to merely detach a tubing section connected with the fluid bag to the tubing section connected with the needle or stent placed intravenously in the patient, which can then be easily connected with a tubing section connected with the new fluid bag. A bayonet tube connector known in the art is disclosed in US 2005/0057042 A1.

Existing tubing connectors are prone to leakage and unwanted disconnection when the patient is still receiving treatment via the connected tubes due to side-loads caused by the weight of the connected tubes and components, as well as accidental pulling of the tubes by the patient or medical personnel.

Furthermore, certain medical devices require the use of multiple tubes for supplying air or fluid between the patient and the device. For example, certain models of blood pressure monitors, such as the Dinamap Procare series, manufactured by General Electric, employ dual tubes for connecting the blood pressure cuff to the monitor. As such, a connector including multiple air passages for directing airflow between the tube segments is desirable, so as to avoid having to individually connect and disconnect multiple connectors when hooking or unhooking a patient to the monitor.

The information included in this Background section of the specification, including any references cited herein and any description or discussion thereof, is included for technical reference purposes only and is not to be regarded subject matter by which the scope of the invention is to be bound.

### SUMMARY

A male bayonet connector may include a shaft defining a lumen therethrough and a grip that facilitates gripping of the shaft by the user. The outer surface of the shaft may define a tubing coupling for connecting with a section of tubing, an annular recess or channel that interfaces with a latch in a female connector for connecting the male bayonet connector with the female connector, and a sealing portion that engages a seal member or surface on an inner diameter of a receiving lumen within the female latch connector for creating a fluid seal between the male and female connectors.

In one implementation, a male bayonet connector includes a shaft defining a lumen and having a distal end portion and a proximal end portion. The proximal end portion of the shaft is configured to engage a section of tubing and the distal end portion of the shaft includes a sealing surface configured to engage a seal member on an inner diameter of a receiving lumen in the female latch connector to create a fluid-tight seal. The male bayonet connector further includes a grip that extends around at least a portion of the shaft. The shaft defines an annular recess proximal to and adjacent the distal end portion. The annular recess has a smaller diameter than the outer diameter of the sealing surface of the distal end portion. The annular recess has a proximal chamfered sidewall and a distal sidewall perpendicular to the axis of the lumen of the shaft. A ratio of a length of the sealing surface to a distance the grip and the distal sidewall is such that a side-load force of up to 68,95 kpa (10 lbs), as imparted on the male bayonet connector, will not break the fluid-tight seal between the sealing surface on the distal end of the shaft and the inner surface of the female receiving structure.

In another implementation, the perpendicular sidewall of the annular channel of the male bayonet connector defines a surface for interfacing with a latch structure within the female receiving structure that resists removal of the male bayonet connector from the female connector.

In another embodiment, the grip extends axially away from the shaft so as to define a flange around the shaft. The flange may define an outer edge and the grip may include a plurality of indentations along the outer edge of the flange for facilitating gripping of the grip.

In other embodiments, the beveled sidewall of the annular channel further defines an angle that is substantially 45 degrees with respect to an axis of the lumen of the shaft. In another embodiment, the ratio of the length of the sealing surface to the distance from the perpendicular sidewall to the grip may be between 0.889 and 1.105.

In another implementation, the male bayonet connector includes two parallel shafts each defining separate lumen and held together by the grip that extends around and between both shafts. The distance between the central axes of the lumen of the parallel shafts may be between 1.695 to 2.035 times the distance from the perpendicular sidewall to the grip.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. A more extensive presentation of features, details, utilities, and advantages of the present invention is provided in the following written description of various embodiments of the invention, illustrated in the accompanying drawings, and defined in the appended claims.

### BRIEF DESCRIPTION OF THE DRAWINGS

FIG. 1 is a front isometric view of a male dual bayonet connector, a female latch connector, and tube sections.
FIG. 2 is a rear isometric view of the male dual bayonet connector shown in FIG. 1.
FIG. 3 is a front elevation view of the male dual bayonet connector shown in FIG. 1.
FIG. 4 is a top plan view of the male dual bayonet connector shown in FIG. 1.
FIG. 5 is a rear elevation view of the male dual bayonet connector shown in FIG. 1.
FIG. 6 is a side elevation view of the male dual bayonet connector shown in FIG. 1.
FIG. 7 is a side elevation view of the male dual bayonet connector, as connected to a female latch connector shown in FIG. 1.
FIG. 8 is a rear isometric view of the male dual bayonet connector and female latch connector in cross section of the connecting member taken along line 8-8 of FIG. 1.
FIG. 9 is an exploded rear isometric view of the female latch connector shown in FIG. 1.
FIG. 10 is a front isometric view of a latch structure of the female latch connector.
FIG. 11 is a side elevation view in cross-section of another embodiment of the male dual bayonet connector.
FIG. 12 is a side elevation view in cross-section of another embodiment of the male dual bayonet connector.
FIG. 13 is a side elevation view in cross-section of another embodiment of the male dual bayonet connector.
FIG. 14 is a rear isometric view of a male bayonet connector and female latch connector.
FIG. 15 is a rear isometric view of a male bayonet connector.

### DETAILED DESCRIPTION

Male bayonet connectors, in conjunction with female latch connectors, may be used to releasably connect sections of tubing. In one embodiment, the male bayonet connector may have a single shaft portion defining a single lumen therethrough and an outer sealing surface that is configured to engage an inner surface of a female latch connector to form a gas and/or liquid fluid seal between the male and female components. The female latch connector may include a latching mechanism that engages a portion of the male bayonet connector so as to prevent removal of the male bayonet connector when connected with the female connector. In another embodiment, the male bayonet connector may have dual shafts, each defining a lumen therethrough. In alternative embodiments, the male bayonet connector may have three or more shafts defining three or more lumen. In embodiments of multiple lumen male bayonet connectors, a grip portion may be used to join the shaft portions, as well as tubing couplings that are configured to engage and retain multiple sections of tubing.

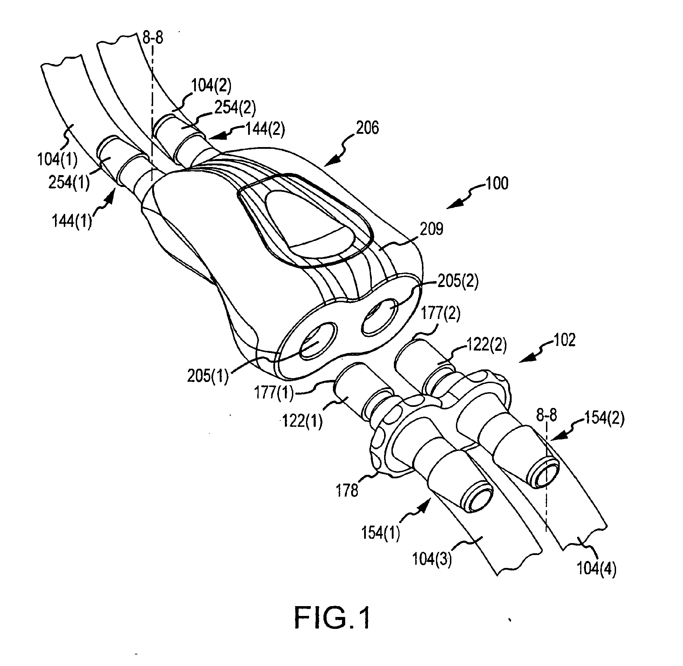

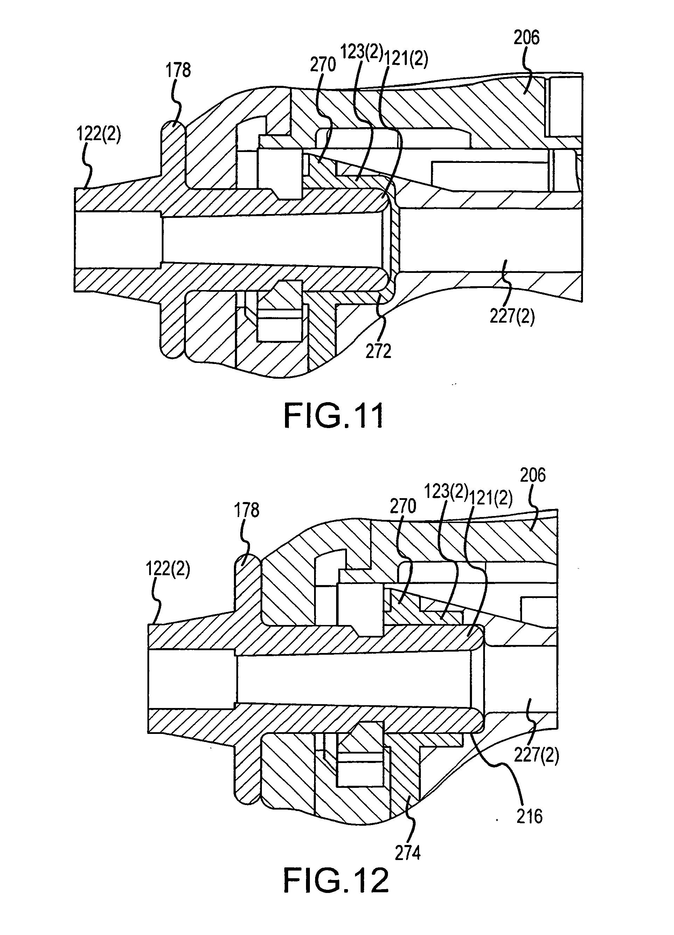

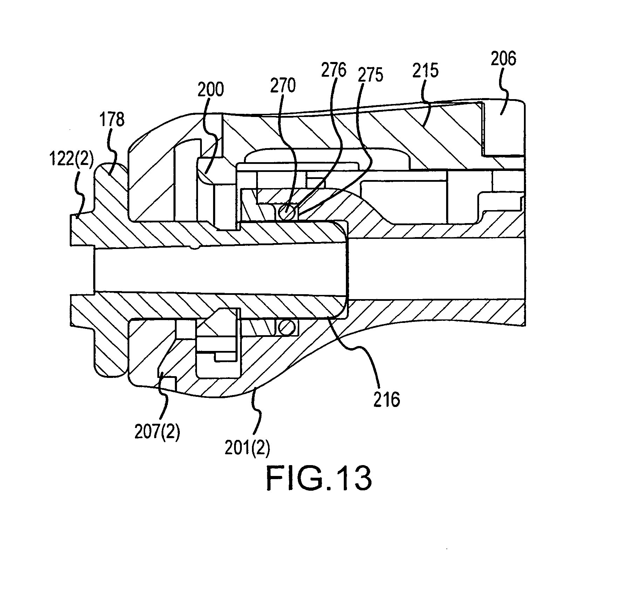

An examplary environment for a male dual bayonet connector 102 is illustrated in FIG. 1. The environment may include a releasable connection assembly 100 and tubing sections 104(1)-104(4). The releasable connection assembly 100 may include the male dual bayonet connector 102 and a female latch connector 206. The male dual bayonet connector 102 may be connected with the female latch connector 206 as will be described further with respect to FIGS. 11-13.

Referring to FIG. 1, first and second tubing sections 104(1)-104(2) may connect with respective tubing couplings 144(1)-144(2) on the distal end of the female latch connector 206. Third and fourth tubing sections 104(3)-104(3) may connect with respective tubing couplings 154(1)-154(2) on the proximal end of the male dual bayonet connector 102. As will be described further below, the male dual bayonet connector 102 may be connected with the female latch connector 206 by inserting the distal end of the male dual bayonet connector 102 into receiving openings 205(1)-205(2) defined in the proximal end of the female latch connector 206. The orientations "proximal" and "distal" as used herein have been arbitrarily chosen, and are not meant to limit the present disclosure, but will follow the convention just described with reference to the ends of the female latch connector 206 and male dual bayonet connector 102.

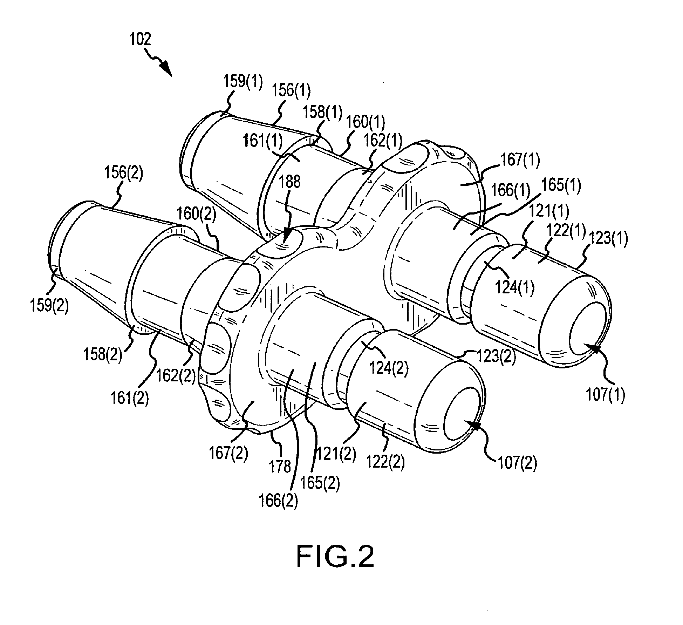

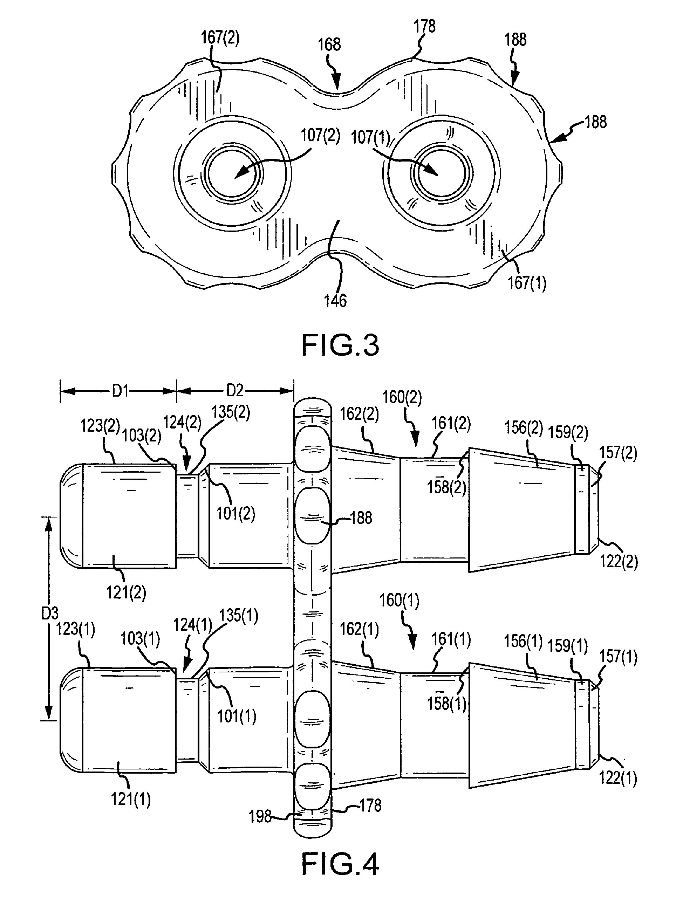

The male dual bayonet connector 102 is illustrated in greater detail in FIGS. 2-6. The male dual bayonet connector 102 may include dual shafts 122(1)-122(2) connected by a grip 178. The dual shafts 122(1)-122(2) may extend from the proximal end of the male bayonet connector 102 to the distal end of the connector 102. As best seen in FIGS. 2, 3 and 5, each of the dual shafts 122(1)-122(2) may define a cylindrical lumen 107(1)-107(2) for transporting fluid from the third and fourth tubing sections 104(3)-104(4) coupled with the male dual bayonet connector 102 to the first and second tubing sections 104(1)-104(2) coupled with the female latch connector 206 via corresponding cylindrical lumens 227(1)-227(2) defined in the female latch connector 206. The dual cylindrical lumens 107(1)-107(2) of the shafts 122(1)-122(2) may be of substantially uniform diameter throughout the entire length of the dual shafts 122(1)-122(2) or, as best shown in cross-section in FIG. 3, may narrow or widen in diameter along the length of the shafts 122(1)-122(2) to include a smaller diameter section and a larger diameter section. In other embodiments, the diameter of the dual cylindrical lumens 107(1)-107(2) may be constant. In further embodiments, the portion of lumens 107(1)-107(2) in the tubing couplings 144(1)-144(2) may be radially offset with respect to the portion of the lumens 107(1)-107(2) along the length of the shafts 122(1)-122(2) to accommodate different tubing configurations. For example, the diameters of the dual cylindrical lumens 107(1)-107(2) may be larger and/or the tubing couplings 144(1)-144(2) may be spaced further or closer apart than the dual shafts 122(1)-122(2) to accommodate varying thicknesses of walls of tubing 104(1)-104(2).

The dual lumen configuration of the male dual bayonet connector 102 allows for simultaneously connecting and disconnecting two or more tubes using a single connection assembly, rather than requiring a separate connection assembly for each tube. As such, the male bayonet connector 102 may provide more efficient connecting and disconnecting of tubes by reducing the amount of time required for medical personnel to hook and unhook a patient from medical equipment.

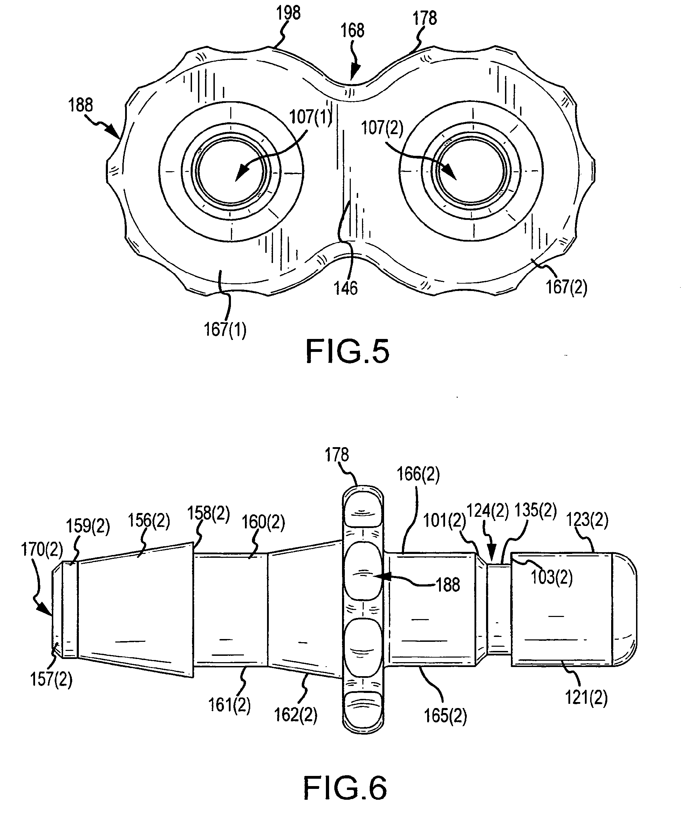

The proximal ends of the dual shafts 122(1)-122(2) may each include a coupling end 156(1)-156(2) shaped as a frustum tapering toward the proximal end for coupling with the third and fourth tube sections 104(3)-104(4) (as seen in FIG. 1). As best seen in FIGS. 4 and 6, the coupling ends 156(1)-156(2) may include a flattened region 159(1)-159(2) toward the proximal ends of the coupling ends 156(1)-156(2), i.e., at the smaller diameter of the frustum. The proximal ends of the coupling ends 156(1)-156(2) may further define a chamfered edge 157(1)-157(2). The exact angle of the chamfered edge 157(1)-157(2) may vary. For example, the chamfered edge 157(1)-157(2) may be between 30-55 degrees. In other embodiments, the proximal ends of the coupling ends 156(1)-156(2) may be rounded or perpendicular to the flattened region 159(1)-159(2). The overall tapered configuration of the coupling ends 156(1)-156(2), including the flattened regions 159(1)-159(2) and chamfered edges 157(1)-157(2) of the coupling ends 156(1)-156(2), may facilitate the insertion of the third and fourth tubing sections 104(3)-104(4) over the coupling ends 156(1)-156(2) of the dual shafts 122(1)-122(2).

The distal ends of the coupling ends 156(1)-156(2), i.e., at the larger diameter of the frustum, may be adjacent to a coupling shaft portion 160(1)-160(2) that may have a first portion 161(1)-161(2) having a narrower outer diameter than that of the distal end of the coupling end 156(1)-156(2), as well as a second portion 162(1)-162(2) that gradually widens in outer diameter toward the grip 178. As such, the coupling shaft portions 160(1)-160(2) may, in some embodiments, vary in outer diameter along the length of the shafts 122(1)-122(2), but in other embodiments, may have a substantially uniform outer diameter that may be narrower than the distal end of the coupling end 156(1)-156(2). The difference in outer diameters between the coupling ends 156(1)-156(2) and the first portions 161(1)-161(2) of the coupling shaft portions 160(1)-160(2) may result in an annular shelf that functions as a coupling barb 158(1)-158(2) for retaining the third and fourth tubing couplings 104(3)-104(4).

The distal ends of the shafts 122(1)-122(2) may define a sealing portion 121(1)-121(2) including a flattened sealing surface 123(1)-123(2). As will be described in further detail below, each sealing surface 123(1)-123(2) may engage a respective sealing member 270 (as shown in, e.g., FIGS. 11-13) in the female latch connector 206 to create a fluid-tight seal between the male dual bayonet connector 102 and the female latch connector 206. The distal end of the sealing portion 121(1)-121(2) may be rounded, as shown in FIGS. 4, 2 and 6, or, in other embodiments, may be chamfered or perpendicular to the sealing surface 123(1)-123(2).

The shafts 122(1)-122(2) may also include proximal portions 165(1)-165(2) defining a proximal shaft portion 166(1)-166(2) that extends toward a grip 178. The proximal shaft portions 166(1)-166(2) may have the same outer diameter as the sealing portion 121(1)-121(2). In one embodiment, the proximal shaft portions 166(1)-166(2) may have a uniform outer diameter. In other embodiments, the proximal shaft portions may have an outer diameter that is either larger or smaller than the outer diameter of the sealing portion 121(1)-121(2).

The shafts 122(1)-122(2) may also each include an annular channel 124(1)-124(2) between the proximal portions 165(1)-165(2) and the sealing portions 121(1)-121(2) that provides for locking of male dual bayonet connector 102 with the female latch connector 206. As shown in FIG. 3, the annular channels 124(1)-124(2) include a bottom region 135(1)-135(2) that has a smaller outer diameter than the outer diameter of the sealing portion 121(1)-121(2). The distal end of each annular channel 124(1)-124(2) is bounded by a distal sidewall 103(1)-103(2) perpendicular to the axes of the cylindrical lumens 107(1)-107(2). The depth of the annular channels 124(1)-124(2) is defined by the difference between the radius of the sealing portion 121(1)-121(2) and the radius of the bottom region 135(1)-135(2).

As best shown in the top and side views of the male dual bayonet connector 102 in FIGS. 4 and 6, the proximal end of each annular recess 124(1)-124(2) may be defined by a chamfered or beveled edge 101(1)-101(2). The beveled edge 101(1)-101(2) may define an angle with respect to the axes of the cylindrical lumens 107(1)-107(2). For example, the surface defined by each beveled edge 101(1)-101(2) may form a 45-degree angle with respect to the axes of the cylindrical lumens 107(1)-107(2). The beveled edge 101(1)-101(2) interfaces with the proximal side of the latch plate 200, which forces the male dual bayonet connector 102 proximally and holds the perpendicular sidewall against the latch plate 200 of the female latch connector 206 in the annular channels 124(1)-124(2). This interface reduces movement of the male dual bayonet connector 102 with respect to the female latch connector 206 and thereby reduces wear of the sealing member 207 in the female latch connector 206. In other embodiments, beveled edges 101(1)-101(2) may be perpendicular to the axes of the cylindrical lumens 107(1)-107(2), may be curved, or alternatively, may define any other angle between 0 and 90 degrees.

The length D1 of the sealing surfaces 123(1)-123(2) of the shafts 122(1)-122(2) as shown in Fig. 3 may bear a relationship to the distance D2 from the perpendicular sidewall 103(1)-103(2) of the annular channels 124(1)-124(2) to the grip 178. In one embodiment, the ratio of the length D1 of the sealing surfaces 123(1)-123(2) to the distance D2 from the perpendicular sidewall 103(1)-103(2) to the grip 178 may be such that a side-load force of up to 10 lbs, as imparted on the male bayonet connector 102, will not break the seal between the sealing surfaces 123(1)-123(2) and the sealing member 270 in the female connector 206. For example, in some implementations, the ratio of the length D1 to the distance D2 may be between 0.889 to 1.105.

A sealing surface 123(1)-123(2) that is proportionally substantially the same or longer with respect to the distance D2 from the annular shelf 103(1)-103(2) to the grip 178 may provide significant lateral support for the shafts 122(1)-122(2) when the male dual bayonet connector 102 is inserted into the female latch connector 206. This proportionality of the length of the shafts 122(1)-122(2) operates to increase resistance to side-load forces and prevent uneven force distribution along the sealing mechanism 270, such as when axial forces are applied to either the male dual bayonet connector 102 or the female latch connector 206. For example, the length of the sealing surface 123(1)-123(2) may allow for better lead-in alignment of the male dual bayonet connector 102 with the female latch connector 206. In addition, the length of the sealing surface 123(1)-123(2), when interfaced with a comparatively long supporting surface within the female latch connector 206, may further resist axial movement of the male dual bayonet connector 102 when connected to the female latch connector 206. The reduction of axial movement of the male dual bayonet connector 102 inside the female latch connector 206 may help resist the sealing member 270 from pinching or slipping off the distal end of the shaft 122(1)-122(2) and sustain contact between the interior surface of the sealing member 270 and the sealing surface 123(1)-123(2) to maintain a fluid-tight seal.

The length of the sealing surface 123(1)-123(2) further allows for positioning the sealing member 270 away from the distal end of the shaft 122(1)-122(2), so as to prevent the sealing member 270 from slipping off of the distal end of the shaft 122(1)-122(2) during engagement of the male dual lumen connector 102 with the female latch connector 206. For example, when interfacing with a supporting region in the female latch connector 206 that extends past the sealing member 270 toward the distal end of the shafts 122(1)-122(2), the engagement of the sealing surface 123(1)-123(2) and the female supporting region may resist axial misalignment of the shafts 122(1)-122(2) under side-loading, thus significantly reducing the possibility of generating a leak path. This serves as an improvement over bayonet designs where the majority of axial support for the shafts is provided at the distal end of the male connector, making these designs much more susceptible to axial and side-loading. Accordingly, the length D1 of the sealing portions 121(1)-121(2) of the shafts 122(1)-122(2) may be selected so as to optimize the stability of the male dual bayonet connector 102 when connected with the female latch connector206.

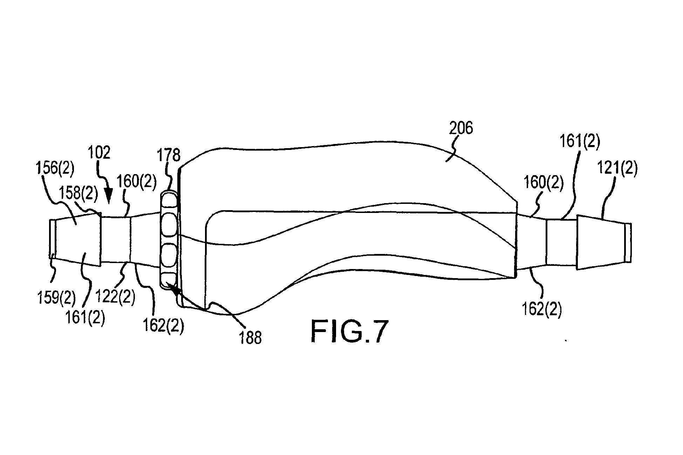

The male dual bayonet connector 102 may also include a grip 178, a portion of which may extend between the dual shafts 122(1)-122(2) to connect the shafts 122(1)-122(2) of the connector 102. In one embodiment, as best shown in FIGS. 7-11, the grip 178 includes two generally circular flanges 167(1)-167(2) that surround the shafts 122(1)-122(2) and that are concentric with the axes of the cylindrical lumens 107(1)-107(2). The flanges 167(1)-167(2) may be connected via a webbed portion 146 formed between the flanges 167(1)-167(2), and may have a larger outer diameter than the other portions of the shafts 122(1)-122(2) of the male dual bayonet connector 102. As such, the grip 178 may function as a stop for preventing over-insertion of the shafts 122(1)-122(2) into the female latch connector 206, and further as a guide for ensuring that the shafts 122(1)-122(2) are fully inserted into the female latch connector 206.

As illustrated in FIGS. 7, 8 and 10, the webbed portion 146 may define a recessed area 168 between the flanges 167(1)-167(2) to allow for easy gripping of the male dual bayonet connector 102 when manipulated by a user. In addition to improving the grip of the male dual bayonet connector 102, providing a recessed area 168 in the webbed portion 146 between the flanges 167(1)-167(2) may further serve to reduce the amount of material required to manufacture the grip 178, thereby decreasing the overall cost associated with manufacturing the male dual bayonet connector 102. In alternate embodiments, there may not be a recessed area between the flanges 167(1)-167 (2) and the perimeter of the grip 178 may be in the form of an oval track with flat sidewalls.

The webbed portion 146 also provides a further benefit, in that it allows for optimal positioning of the lumens 107(1)-107(2) of the male dual bayonet connector 102 with respect to one another. In particular, the webbed portion 146 allows for positioning of the lumens 107(1)-107(2) so that the space between the central axes of the lumens 107(1)-107(2) can be maximized to allow for convenient connection and removal of both individual and webbed tubes, i.e., tubes connected with an intermediate web along their length, without modifying the tubing. In one embodiment, a distance D3 between the axes of the lumens 107(1)-107(2) may be between approximately 1.695 to 2.035 times the length D2 between the perpendicular sidewall 103(1)-103(2) and the grip 178. Additionally, a wider webbed portion 146 may position the lumens 107(1)-107(2) further apart and may help prevent tangling of the attached tubing, while a narrower webbed portion 146 would position the lumens 107(1)-107(2) closer together. A wider webbed portion 146 may alternatively allow for thicker-walled tubing to be attached to the male dual bayonet connector 102 by providing sufficient clearance for thicker tube walls. Accordingly, the width of the webbed portion 146 may be varied according to the specifications of the tubing being attached to the male dual bayonet connector 102.

Additionally, the outer edge 198 of the grip 178 may include one or more evenly-spaced indentations 188 to further facilitate gripping of the male dual bayonet connector 102 by a user. In the embodiment illustrated in FIGS. 2-6, the outer edge 198 of the grip 178 includes twelve (12) evenly-spaced indentations 188, with each ring 167(1)-167(2) including six (6) indentations 188, and a recessed webbed portion 146 extending between the rings 167(1)-167(2). However, the exact number, shape, and size of the indentations 188 is not critical so long as the grip 178 provides an enhanced gripping surface for the user. As such, in other embodiments, the number, shape, and size of the indentations 188 along the grip 178 may vary.

Another function of the grip 178 is to provide proper lead-in alignment of the male dual bayonet connector 102 with the female latch connector 206, thereby allowing for proper insertion of the male dual bayonet connector 102 into the female latch connector 206. Furthermore, the grip 178 ensures axial alignment of the shafts 122(1)-122(2) with the receiving openings 205(1)-205(2) of the female latch connector 206 during engagement, so as to allow for even distribution of the pressure applied by the sealing surface 123(1)-123(2) against the sealing member 270 to prevent leakage around the sealing surface 123(1)-123(2), as well as deformation and/or uneven wearing of the sealing member 270 over time.

In a further embodiment, a flat rib (not shown) may extend between the proximal portions 165(1)-165(2) of the shafts 122(1)-122(2) to provide greater structural rigidity to the male dual lumen connector 102. The length and thickness of the rib may vary depending upon design requirements or constraints or with the relative durometer of the material used to form the connector 102. The rib may or may not be connected to the webbed portion 146 of the grip 178.

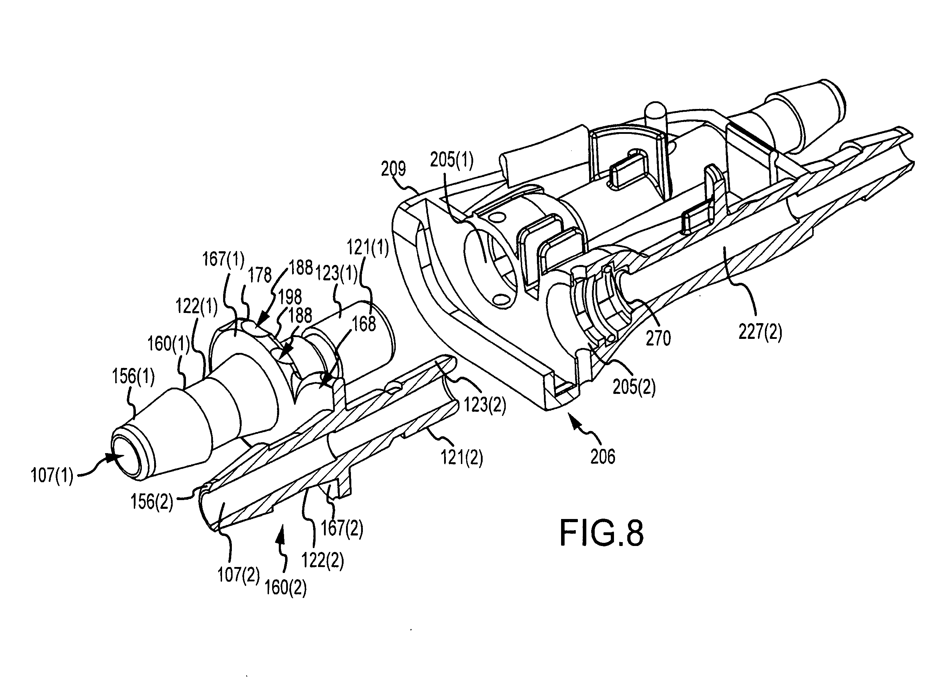

One embodiment of a female latch connector 206 that may be connected to the male dual bayonet connector 102 is illustrated in FIGS. 8-10. The female latch connector 206 may include an exterior enclosure 209 defining two openings 205(1)-205(2) for receiving the dual shafts 122(1)-122(2) of the male dual bayonet connector 102. As shown in FIG. 7, the female latch connector 206 may further include a latch plate structure 200 defining two receiving apertures 203(1)-203(2) that are axially aligned with the exterior openings 205(1)-205(1) of the assembled female latch connector 206, so as to receive the shafts 122(1)-122(2).

The female latch connector 206 may further define two cylindrical lumens 227(1)-227(2) that extend through the female latch connector 206. In one embodiment, the cylindrical lumens 227(1)-227(2) of the female latch connector 206 are positioned so that when the female latch connector 206 and the male dual lumen connector 102 are connected, the female lumens 227(1)-227(1) are axially aligned with at least a portion of the male cylindrical lumens 107(1)-107(2) to facilitate fluid flow between the connected male and female connectors 102 and 206. In other embodiments, sections of the lumens 107(1)-107(2) and 227(1)-227(2) of the male 102 or female 206 connectors may be offset with respect to one another. Additionally, the female latch connector 206 may include two tubing couplings 254(1)-254(2) that are each configured to engage a section of tubing 104(1)-104(2), as shown in FIG. 1. The tubing couplings 254(1)-254(2) of the female latch connector 206 may be similar in configuration to the male tubing couplings 156(1)-156(2)

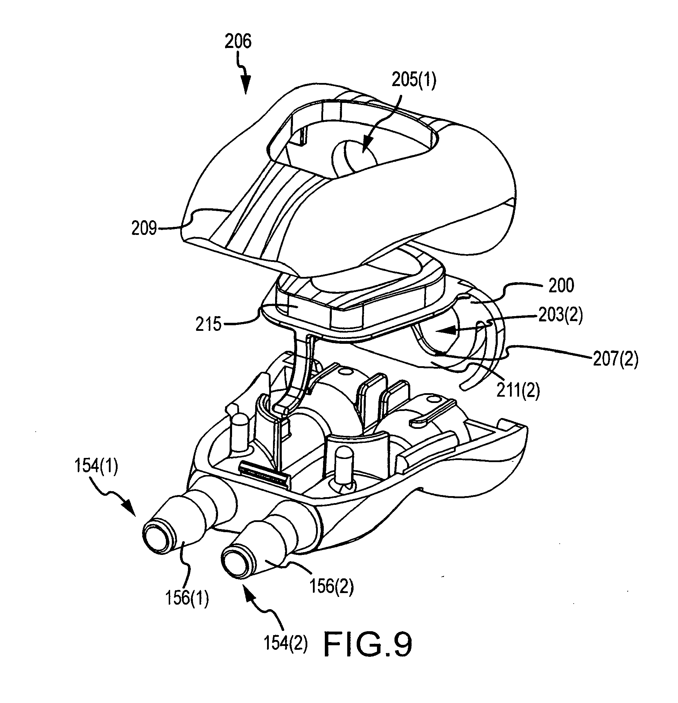

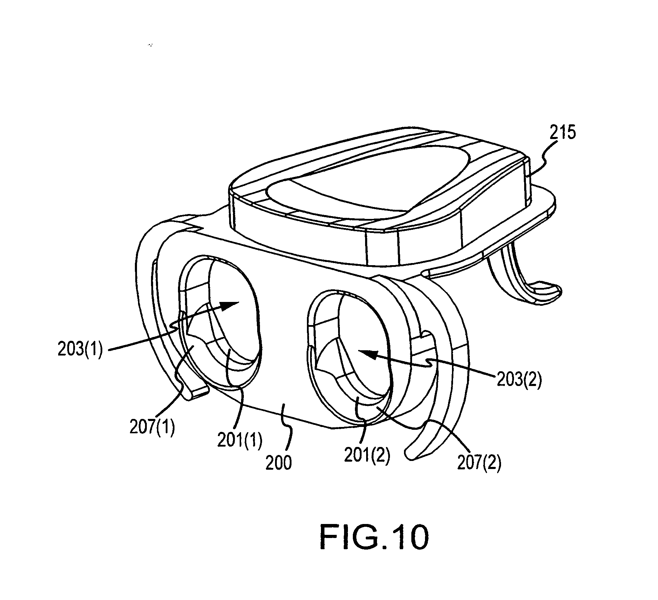

The latch plate structure 200 of the female latch connector 206 is shown in FIGS. 9 and 10. As best seen in FIG. 10, latching surfaces 201(1)-201(2) may be formed along the bottom walls of the receiving apertures 203(1)-203(2) of the latch plate structure 200. In one embodiment, the latch plate 200 may be resiliently biased upward to lift the latching surfaces 201(1)-201(2) so as to interface with the annular channels 124(1)-124(2) in the male dual bayonet connector 102. For example, as the shafts 122(1)-122(2) are inserted through the apertures 203(1)-203(2), the latch plate 200 may be biased downward to lower the receiving apertures 203(1)-203(2) to accommodate the outer diameter of the shafts 122(1)-122(2). In one embodiment, the receiving apertures 203(1)-203(2) may each be defined by a chamfered edge 207(1)-207(2) that is angled to facilitate the insertion of the shafts 122(1)-122(2) through the receiving apertures 203(1)-203(2) of the latch structure 200. The distal face of the latch plate 200 may define distal latching edges 211(1)-211(2) that may interface with the annular shelves 103(1)-103(2) of the shafts 122(1)-122(2) to prevent the shafts 122(1)-122(2) from being removed from the female latch connector 206.

The latch surfaces 201(1)-201(2) may be operably coupled to a release mechanism 215 for disengaging the latch surfaces 201(1)-201(2) from the male dual bayonet connector 102. For example, as shown in FIGS. 1, 9 and 10, the release mechanism 215 may be a button that, when depressed, may lower the latch plate 200 so that the latch surfaces 201(1)-201(2) may clear the annular channels 124(1)-124(2), allowing for removal of the shafts 122(1)-122(2) from the receiving apertures 205(1)-205(2) of the female latch connector 206.

The female latch connector 206 may further include a sealing member 270 that engages the sealing surface 123(1)-123(2) of the dual shafts 122(1)-122(2) to form a fluid-tight seal between the female receiving portion 206 and the male dual bayonet connector 102. The sealing member 270 may be made from an elastomeric material that may enhance the sealing interface between the female sealing member 270 and the sealing surface 123(1)-123(2) of the male dual bayonet connector 102.

As best shown in FIGS. 11-13, illustrating the female latch connector 206 connected to the male dual bayonet connector 102, the configuration of the sealing member 270 within the female receiving portion 206 may vary according to different embodiments of the female receiving portion 206. As shown in FIG. 9, in one embodiment, the sealing member 270 may be an over-molded seal 272 that extends along the entire length D1 of the sealing portion 121(1)-121(2) so as to cover the entire sealing surface 123(1)-123(2), as well as forming and end seal with the distal end of the male dual bayonet connector 102. In another embodiment, illustrated in FIG. 10, the sealing member 270 may be an over-molded seal 274 that may cover only a portion of the length D1 of the sealing surface 123(1)-123(2). In yet another embodiment, illustrated in FIG. 11, the sealing member 270 may include an O-ring 276 that has a point contact with the sealing surface 123(1)-123(2). The O-ring 276 may be seated within a recessed area 275 defined by the female latch connector 206.

To connect the male dual bayonet connector 102 with the female latch connector 206, the dual shafts 122(1)-122(2) may be inserted through the openings 205(1)-205(2) (shown in FIG. 1) defined in the exterior enclosure 209 of the female latch connector 206 and the apertures 203(1)-203(2) defined by the latch plate structure 200. Insertion of the shafts 122(1)-122(2) through the apertures 203(1)-203(2) of the latch plate structure 200 causes the latch plate 200 to lower due to the interaction between the rounded distal ends 177(1)-177(2) of the shafts 122(1)-122(2) and the chamfered edges 207(1)-207(2) of the latch surfaces 201(1)-201(2).

Once the shafts 122(1)-122(2) are inserted far enough so that the latch surfaces 201(1)-201(2) are positioned below the annular channels 124(1)-124(2), the latch plate structure 200 may lift so that at least a portion of the latch surfaces 201(1)-201(2) is at least partially seated within the annular channels 124(1)-124(2). As best shown in cross section in FIGS. 9-11, the beveled edges 101(1)-101(2) defined by the annular channels 124(1)-124(2) may be angled to oppose the angle defined by the chamfered edges 207(1)-207(2) of the latch surfaces 201(1)-201(2), thereby preventing lateral movement of the male dual bayonet connector 102 with respect to the connected female latch connector 206.

The distal latching edges 211(1)-211(2) of the latch plate 200 may interface with the perpendicular distal sidewall 103(1)-103(2) of the annular channels 124(1)-124(2) so as to prevent removal of the shafts 122(1)-122(2) from the female receiving portion 206. The perpendicular distal sidewalls 103(1)-103(2) resist disengagement from the latch plate 200 under longitudinal and axial loads. In one embodiment, the distal latching edges 211 (1)-211(2) of the latch face 200 may oppose the perpendicular distal sidewalls 103(1)-103(2) defined in the shafts 122(1)-122(2) to provide a greater axial retention force, as well as the ability to lock the male dual bayonet connector 102 with the female receiving portion 206 from the bottom of the shafts 122(1)-122(2), as opposed to the sides of the shafts 122(1)-122(2). This bottom locking feature further lessens the distance required for lowering the latch plate 200 to release the male dual bayonet connector 102, thereby improving the overall ergonomic design of the female latch connector 206 and minimizing the insertion force required for inserting the male dual bayonet connector 102 into the female latch connector 206.

The elongated sealing surface 123(1)-123(2) of the shafts 122(1)-122(2) may allow for positioning of the sealing mechanism 270 away from the distal end of the sealing surface 123(1)-123(2). As discussed above, this may help prevent the sealing mechanism 270 from pinching or slipping off from the distal end of the shaft 122(1)-122(2), and to sustain contact between the interior surface of the sealing mechanism 270 and the sealing surface 123(1)-123(2) to maintain a fluid-tight seal when axial forces are applied to either of the connected the male dual bayonet connector 102 or the female latch connector 206. In some embodiments, such as when the female latch connector 206 includes an O-ring 276 or partial molded seal 274, the female latch connector 206 may include an additional supporting surface 216 that is positioned around the distal end of the shafts 122(1)-122(2) for providing additional axial support for the shafts 122(1)-122(2), and further preventing deformation of the sealing mechanism 270.

To remove the male dual bayonet connector 102 from the female receiving portion 206, a user may depress the release mechanism 215 to lower the latch plate 200 until the latch surfaces 201(1)-201(2) clear the annular channels 124(1)-124(2). Once the annular channels 124(1)-124(2) are cleared, the male dual bayonet connector 102 may be easily disengaged from the female latch connector 206.

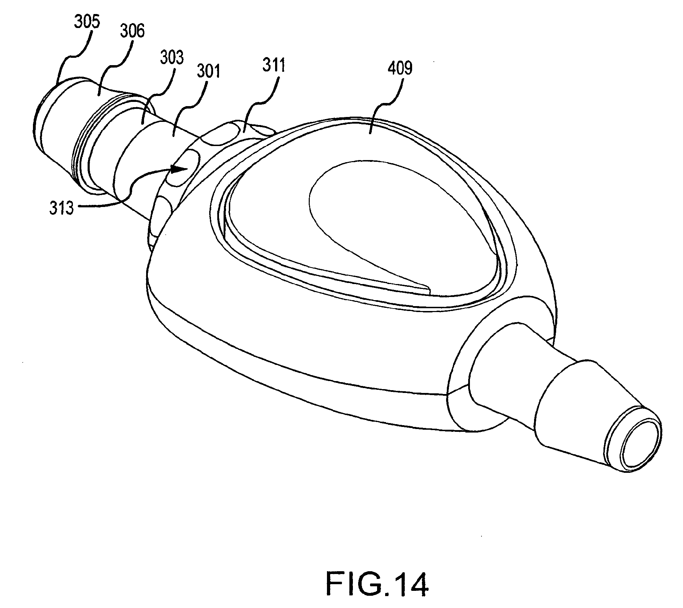

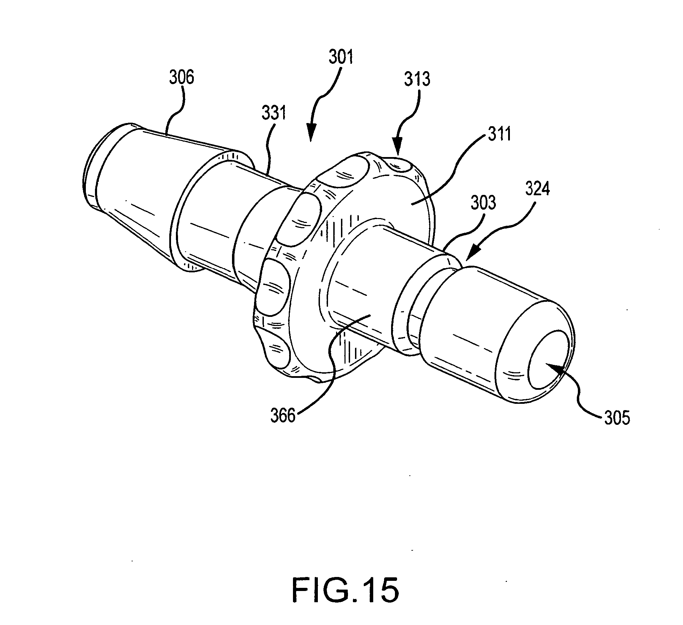

FIGS. 14 and 15 illustrate a single-lumen embodiment of the male bayonet connector 301. In this embodiment, the male bayonet connector 301 may include a single shaft 303, and a single lumen 305 extending through the length of the shaft 303. Similar to the male dual bayonet connector 102, the proximal end of the male bayonet connector 301 may include a coupling end 306 for connecting with a tubing section. The tubing coupling 306 may have a configuration similar to the coupling ends 156(1)-156(2) of the male dual bayonet connector 102 illustrated in FIGS. 2-6. The shaft 303 may further include a coupling shaft portion 331 similar to the coupling shaft portions 160(1)-160(2) of the male bayonet connector 102, and a proximal shaft portion 366 similar in configuration to the proximal shaft portions 166(1)-166(2) of the connector 102. The distal end of the male bayonet connector 301 may include a sealing surface similar to the flattened sealing surface 123(1)-123(2) of the male dual bayonet connector 102 illustrated in FIGS. 2-6, as well as an annular channel 324 that is configured similar to the annular channels124(1)-124(2) of the male dual bayonet connector 102. In addition, the male bayonet connector 301 may include a ring-shaped grip 311 including a plurality of indentations 313 along the outer edge thereof. In one embodiment, the grip 311 of the male bayonet connector 301 may include ten (10) indentations.

As shown in FIG. 14, the female connector 409 may include a latch plate structure that is similar to the latch plate structure 200 of the female latch connector 206. The latch plate of the female connector 209 may include a single aperture for receiving the shaft 303 of the male bayonet connector 301. Additionally, the female connector 209 may include a sealing mechanism similar to the sealing mechanisms 270 shown in FIGS. 9-11. When connected, the latch plate may interface with the annular channel of the shaft 303, and the sealing mechanism may interface with the male sealing surface in a manner similar to that previously described with respect to FIGS. 11-13. Other embodiments of male bayonet connectors and female latch connectors may include any number of lumens, barbs, and associated shaft portions, as appropriate for the medical procedure being performed.

It will be apparent to those of ordinary skill in the art that variations and alternative embodiments may be made given the foregoing description. Such variations and alternative embodiments are accordingly considered within the scope of the present invention.

As used herein, lumen refers not only to its definition, but also refers to an opening, aperture, or other passageway. The fluid referred to herein can be gaseous, liquid, or other state of material that is flowable through a tube (i.e., granular). In addition, while generally described above as sealed when connected together, the connector structures may be sealed or unsealed. The connection between the male dual bayonet connector and female latch connectors and their respective tube sections can be by means other than a barbed fitting, for example, but not limited to, threaded, press-fit without a barb, John Guest fitting, ferrule, and panel mount.

All directional references (e.g., upper, lower, upward, downward, left, right, leftward, rightward, top, bottom, above, below, inner, outer, vertical, horizontal, clockwise, and counterclockwise) are only used for identification purposes to aid the reader's understanding of the example of the invention, and do not create limitations, particularly as to the position, orientation, or use of the invention unless specifically set forth in the claims. Joinder references (e.g., attached, coupled, connected, joined, and the like) are to be construed broadly and may include intermediate members between a connection of elements and relative movement between elements. As such, joinder references do not necessarily infer that two elements are directly connected and in fixed relation to each other.

In some instances, components are described with reference to "ends" having a particular characteristic and/or being connected with another part. However, those skilled in the art will recognize that the present invention is not limited to components which terminate immediately beyond their points of connection with other parts. Thus, the term "end" should be interpreted broadly, in a manner that includes areas adjacent, rearward, forward of, or otherwise near the terminus of a particular element, link, component, part, member or the like. In methodologies directly or indirectly set forth herein, various steps and operations are described in one possible order of operation, but those skilled in the art will recognize that steps and operations may be rearranged, replaced, or eliminated without necessarily departing from the scope of the present invention.

The above specification, examples and data provide a complete description of the structure and use of exemplary embodiments of the invention. Although various embodiments of the invention have been described above with a certain degree of particularity, or with reference to one or more individual embodiments, those skilled in the art could make numerous alterations to the disclosed embodiments without departing from the scope of this invention. Other embodiments are therefore contemplated. It is intended that all matter contained in the above description and shown in the accompanying drawings shall be interpreted as illustrative only of particular embodiments and not limiting. Changes in detail or structure may be made without departing from the basic elements of the invention as defined in the following claims.

## Claims

1. A male bayonet connector (102, 301) for connecting sections of tubing (104(1) - 104(4)), comprising
a first shaft (122(1)) having a first distal end portion and a first proximal end portion and defining a first lumen (107(1)) and a first annular channel (124(1)) proximal to and adjacent the first distal end portion; and
a grip (178) extending around at least a portion of the first shaft (122(1)); wherein
the first proximal end portion of the first shaft (122(1)) is configured to engage a first section of tubing (104(1)) and the first distal end portion of the first shaft (122(1)) has a first sealing surface (123(1)) configured to engage a first inner surface of a female receiving structure (206) to create a fluid-tight seal;
the first annular channel (124(1)) is defined by a distal sidewall (103(1));
**characterized in that** a ratio of a length (D1) of the first sealing surface (123(1)) to a distance (D2) between the distal sidewall (103(1)) and the grip (178) is between 0.889 and 1.105 such that a side load force of up to 68.95 kPa (10 lbs), as imparted on the male bayonet connector (102, 301), will not break the fluid-tight seal between the first sealing surface (123(1)) and the first inner surface of the female receiving structure (206).

2. The male bayonet connector (102, 301) of claim 1, wherein the first distal sidewall (103(1)) defines a surface that is oriented substantially perpendicular to an axis of the first lumen (107(1)).

3. The male bayonet connector (102, 301) of claim 1, wherein the first distal sidewall (103(1)) defines a surface for interfacing with a first latch structure (200) within the female receiving structure (206) so as to prevent removal of the male bayonet connector (102, 301) from the female receiving structure (206) when the first latch structure (200) engages the first annular channel (124(1)).

4. The male bayonet connector (102, 301) of claim 1, wherein the grip (178) extends axially away from the first shaft (122(1)) so as to define a flange (167(1)) around the first shaft (122(1));
and wherein preferably the flange (167(1)) defines an outer edge and the grip (178) includes a plurality of indentations (188) along the outer edge of the flange (167(1)) for facilitating gripping of the grip (178).

5. The male bayonet connector (102, 301) of claim 1, wherein the first annular channel (124(1)) further defines a first beveled edge (101(1)) opposite the first distal sidewall (103(1));
and wherein preferably a surface of the first beveled edge (101(1)) defines an angle that is substantially 45 degrees with respect to an axis of the first lumen (107(1)).

6. The male bayonet of claim 1, wherein the grip (178) is positioned to prevent further insertion of the first shaft (122(1)) into the female receiving structure (206).

7. The male bayonet connector (102, 301) of claim 1, further comprising
a second shaft (122(2)) having a second distal end portion and a second proximal end portion and further defining a second lumen (107(2)) and a second annular channel (124(2)) proximal to and adjacent the second distal end portion, wherein
the second proximal end portion of the second shaft (122(2)) is configured to engage a second section of tubing (104(2)) and the second distal end portion of the second shaft (122(2)) has a second sealing surface (123(2)) configured to engage a second inner surface of a corresponding female receiving structure (206) to create a fluid-tight seal;
the second annular channel (124(2)) is defined by a second distal sidewall (103(2)); and
a ratio of a length (D1) of the second sealing surface (123(2)) to a distance (D2) between the second distal sidewall (103(2)) and the grip (178) is such that a side load force of up to 68.95 kPa (10 lbs), as imparted on the male bayonet connector (102, 301), will not break the fluid-tight seal between the second sealing surface (123(2)) and the second inner surface of the female receiving structure (206).

8. The male bayonet connector (102, 301) of claim 7, wherein the grip (178) connects the first and second shafts (122(1), 122(2));
wherein preferably the grip (178) extends axially away from the first shaft (122(1)) so as to define a first flange (167(1)) around the first shaft (122(1)) and axially away from the second shaft (122(2)) so as to define a second flange (167(2)) around the second shaft (122(2)), and the first and second flanges (167(1), 167(2)) are connected by a webbed portion therebetween; and
wherein in particular the webbed portion defines a recessed area between the first and second flanges (167(1), 167(2)).

9. A male bayonet connector (102, 301) for connecting of claim 1, further comprising:
a second shaft (122(2)) having a second distal end portion and a second proximal end portion and further defining a second lumen (107(2)), wherein the second proximal end portion of the second shaft (122(2)) is configured to engage a second section (104(2)) of tubing and the second distal end portion of the second shaft (122(2)) has a second sealing surface (123(2)) configured to engage a second inner surface of a female receiving structure (206) to create a fluid-tight seal; and
the grip (178) connecting the first and second shafts (122(1), 122(2)); wherein
the first distal sidewall (103(1)) is perpendicular with respect to an axis of the first lumen (107(1)) and a first proximal sidewall having a beveled edge (101(1)).

10. The male bayonet connector (102, 301) of claim 9, wherein
the second shaft (122(2)) defines a second annular channel (124(2)) proximal to and adjacent the second distal end portion,
and further defines a second annular shelf defining a distal wall of the second annular channel (124(2)) and a second beveled edge (101(2)) defining a proximal wall of the second annular channel (124(2)),
the second annular shelf defines a surface that is perpendicular with respect to an axis of the second lumen (107(2)), and
a ratio of a length (D1) of the second sealing surface (123(2)) to a distance (D2) from the second annular shelf to the grip (178) is such that a side load force of up to 68.95 kPa (10 lbs), as imparted on the male bayonet connector (102, 301), will not break the fluid-tight seal between the second sealing surface (123(2)) and the second inner surface of the female receiving structure (206).

11. The male bayonet connector (102, 301) of claim 9, wherein the grip (178) extends axially away from the first and second shafts (122(1), 122(2)) so as to define a first flange (167(1)) around the first shaft (122(1)) and a second flange (167(2)) around the second shaft (122(2)).

12. The male bayonet connector (102, 301) of claim 10, wherein the first and second shafts (122(1), 122(2)) are connected by a webbed portion that extends between the first and second flanges (167(1), 167(2)).

13. The male bayonet of claim 7 or 9, wherein a distance (D2) between a first central axis of the first lumen (107(1)) and a second central axis of the second lumen (107(2)) is between 1.695 to 2.035 times the distance (D2) from the first distal sidewall (103(1)) to the grip (178).

## Patentansprüche

1. Ein männlicher Bajonettverbinder (102, 301) zum Verbinden von Rohrsektionen (104(1)- 104(4)), umfassend
einen ersten Schaft (122(1)), welcher einen ersten distalen Endabschnitt und einen ersten proximalen Endabschnitt hat und welcher einen ersten Hohlraum (107(1)) und einen ersten Ringkanal (124(1)) proximal und benachbart zu dem ersten distalen Endabschnitt definiert; und
einen Griff (178), welcher sich zumindest um einen Abschnitt des ersten Schafts (122(1)) herum erstreckt; wobei
der erste proximale Endabschnitt des ersten Schafts (122(1)) dazu eingerichtet ist, an einer ersten Rohrsektion (104(1)) anzuliegen und der erste distale Endabschnitt des ersten Schafts (122(1)) eine erste Dichtungsoberfläche (123(1)) hat, welche dazu eingerichtet ist, an einer ersten inneren Oberfläche einer weiblichen Empfängerstruktur (206) anzuliegen, um eine fluiddichte Dichtung zu erzeugen;
der erste Ringkanal (124(1)) durch eine distale Seitenwand (103(1)) definiert ist;
**dadurch gekennzeichnet, dass** ein Verhältnis einer Länge (D1) der ersten Dichtungsoberfläche (123(1)) zu einem Abstand (D2) zwischen der distalen Seitenwand (103(1)) und dem Griff (178) zwischen 0,889 und 1,105 liegt, sodass eine Seitenbelastungskraft bis zu 68,95 kPa (10 lbs), aufgebracht auf den männlichen Bajonettverbinder (102, 301), nicht zu einem Aufheben der fluiddichten Dichtung zwischen der ersten Dichtungsoberfläche (123(1)) und der ersten inneren Oberfläche der weiblichen Empfängerstruktur (206) führt.

2. Der männliche Bajonettverbinder (102, 301) nach Anspruch 1, wobei die erste distale Seitenwand (103(1)) eine Oberfläche definiert, welche im Wesentlichen senkrecht zu einer Achse des ersten Hohlraums (107(1)) ausgerichtet ist.

3. Der männliche Bajonettverbinder (102, 301) nach Anspruch 1, wobei die erste distale Seitenwand (103(1)) eine Oberfläche zum Koppeln mit einer ersten Riegelstruktur (200) innerhalb der weiblichen Empfängerstruktur (206) definiert, um so die Entnahme des männlichen Bajonettverbinders (102, 301) aus der weiblichen Empfängerstruktur (206) zu verhindern, wenn die erste Riegelstruktur (200) an dem ersten Ringkanal (124(1)) anliegt.

4. Der männliche Bajonettverbinder (102, 301) nach Anspruch 1, wobei der Griff (178) sich axial weg von dem ersten Schaft (122(1)) erstreckt, um so einen Flansch (167(1)) um den ersten Schaft (122(1)) herum zu definieren;
und wobei der Flansch (167(1)) vorzugsweise eine äußere Kante definiert und der Griff (178) eine Mehrzahl von Vertiefungen (188) entlang der äußeren Kante des Flansches (167(1)) zum Vereinfachen des Greifens des Griffes (178) beinhaltet.

5. Der männliche Bajonettverbinder (102, 301) nach Anspruch 1, wobei der erste Ringkanal (124(1)) ferner eine erste schräge Kante (101(1)) gegenüber der ersten distalen Seitenwand (103(1)) definiert;
und wobei eine Oberfläche der ersten schrägen Kante (101(1)) vorzugsweise einen Winkel definiert, welcher im Wesentlichen 45 Grad zu einer Achse des ersten Hohlraums (107(1)) entspricht.

6. Das männliche Bajonett nach Anspruch 1, wobei der Griff (178) positioniert ist, um ein weiteres Einsetzen des ersten Schafts (122(1)) in die weibliche Empfängerstruktur (206) zu verhindern.

7. Der männliche Bajonettverbinder (102, 301) nach Anspruch 1, ferner umfassend
einen zweiten Schaft (122(2)), welcher einen zweiten distalen Endabschnitt und einen zweiten proximalen Endabschnitt hat und ferner einen zweiten Hohlraum (107(2)) und einen zweiten Ringkanal (124(2)) proximal und benachbart zu dem zweiten distalen Endabschnitt definiert, wobei
der zweite proximale Endabschnitt des zweiten Schaftes (122(2)) dazu eingerichtet ist, an einer zweiten Rohrsektion (104(2)) anzuliegen und wobei der zweite distale Endabschnitt des zweiten Schafts (122(2)) eine zweite Dichtungsoberfläche (123(2)) hat, welche dazu eingerichtet ist, an einer zweiten inneren Oberfläche einer korrespondierenden weiblichen Empfängerstruktur (206) anzuliegen, um eine fluiddichte Dichtung zu erzeugen;
der zweite Ringkanal (124(2)) durch eine zweite distale Seitenwand (103(2)) definiert ist; und
ein Verhältnis von einer Länge (D1) der zweiten Dichtungsoberfläche (123(2)) zu einem Abstand (D2) zwischen der zweiten distalen Seitenwand (103(2)) und dem Griff (178) derart ist, dass eine Seitenbelastungskraft bis zu 68,95 kPa (10 lbs), aufgebracht auf den männlichen Bajonettverbinder (102, 301), nicht zu einem Aufheben der fluiddichten Dichtung zwischen der zweiten Dichtungsoberfläche (123(2)) und der zweiten inneren Oberfläche der weiblichen Empfängerstruktur (206) führt.

8. Der männliche Bajonettverbinder (102, 301) nach Anspruch 7, wobei der Griff (178) den ersten und zweiten Schaft (122(1), 122(2)) verbindet;
wobei sich der Griff (178) vorzugsweise axial weg von dem ersten Schaft (122(1)) erstreckt, um so einen ersten Flansch (167(1)) um den ersten Schaft (122(1)) herum zu definieren und axial weg von dem zweiten Schaft (122(2)), um so einen zweiten Flansch (167(2)) um den zweiten Schaft (122(2)) herum zu definieren, und wobei der erste und zweite Flansch (167(1), 167(2)) dazwischen durch einen Stegabschnitt verbunden sind; und
wobei der Stegabschnitt insbesondere einen vertieften Bereich zwischen dem ersten und dem zweiten Flansch (167(1), 167(2)) definiert.

9. Ein männlicher Bajonettverbinder (102, 301) zum Verbinden nach Anspruch 1, ferner umfassend:
einen zweiten Schaft (122(2)), welcher einen zweiten distalen Endabschnitt und einen zweiten proximalen Endabschnitt hat und ferner einen zweiten Hohlraum (107(2)) definiert, wobei der zweite proximale Endabschnitt des zweiten Schafts (122(2)) dazu eingerichtet ist, an einer zweiten Rohrsektion (104(2)) anzuliegen und der zweite distale Endabschnitt des zweiten Schafts (122(2)) eine zweite Dichtungsoberfläche (123(2)) hat, welche dazu eingerichtet ist, an einer zweiten inneren Oberfläche einer weiblichen Empfängerstruktur (206) anzuliegen, um eine fluiddichte Dichtung zu erzeugen; und
wobei der Griff (178) den ersten und zweiten Schaft (122(1), 122(2)) verbindet; wobei
die erste distale Seitenwand (103(1)) senkrecht zu einer Achse des ersten Hohlraums (107(1)) ausgerichtet ist und eine erste proximale Endwand eine schräge Kante (101(1)) hat.

10. Der männliche Bajonettverbinder (102, 301) nach Anspruch 9, wobei
der zweite Schaft (122(2)) einen zweiten Ringkanal (124(2)) proximal und benachbart zu dem zweiten distalen Endabschnitt definiert,
und ferner eine zweite ringförmige Schale, welche eine distale Wand des zweiten Ringkanals (124(2)) definiert, und eine zweite schräge Kante (101(2)) definiert, welche eine proximale Wand des zweiten Ringkanals (124(2)) definiert,
die zweite ringförmige Schale eine Oberfläche definiert, welche senkrecht zu einer Achse des zweiten Hohlraums (107(2)) ausgerichtet ist, und
ein Verhältnis einer Länge (D1) der zweiten Dichtungsoberfläche (123(2)) zu einem Abstand (D2) von der zweiten ringförmigen Schale zu dem Griff (178) derart ist, dass eine Seitenbelastungskraft bis zu 68,95 kPa (10 lbs), aufgebracht auf den männlichen Bajonettverbinder (102, 301), nicht zu einem Aufheben der fluiddichten Dichtung zwischen der zweiten Dichtungsoberfläche (123(2)) und der zweiten inneren Oberfläche der weiblichen Empfängerstruktur (206) führt.

11. Der männliche Bajonettverbinder (102, 301) nach Anspruch 9, wobei der Griff (178) sich axial weg von dem ersten und zweiten Schaft (122(1), 122(2)) erstreckt, um so einen ersten Flansch (167(1)) um den ersten Schaft (122(1)) herum und einen zweiten Flansch (167(2)) um den zweiten Schaft (122(2)) herum zu definieren.

12. Der männliche Bajonettverbinder (102, 301) nach Anspruch 10, wobei der erste und zweite Schaft (122(1), 122(2)) durch einen Stegabschnitt, welcher sich zwischen dem ersten und zweiten Flansch (167(1), 167(2)) erstreckt, verbunden sind.

13. Das männliche Bajonett nach Anspruch 7 oder 9, wobei ein Abstand (D2) zwischen einer ersten Mittelachse des ersten Hohlraums (107(1)) und einer zweiten Mittelachse des zweiten Hohlraums (107(2)) zwischen 1,695 bis 2,035 mal dem Abstand (D2) von der ersten distalen Seitenwand (103(1)) zu dem Griff (178) beträgt.

## Revendications

1. Connecteur à baïonnette mâle (102, 301) pour la liaison de sections de tubes (104(1) - 104(4)), comprenant
un premier arbre (122(1)) présentant une première partie d'extrémité distale et une première partie d'extrémité proximale et définissant un premier lumen (107(1)) et un premier canal annulaire (124(1)) proximal et adjacent à la première partie d'extrémité distale ; et
une poignée (178) s'étendant autour d'au moins une partie du premier arbre (122(1)) ; dans lequel
la première partie d'extrémité proximale du premier arbre (122(1)) est configurée pour être en prise avec une première section de tubes (104(1)) et la première partie d'extrémité distale du premier arbre (122(1)) présente une première surface étanche (123(1)) configurée pour être en prise avec une première surface intérieure d'une structure de réception femelle (206) pour créer un joint étanche au fluide ;
le premier canal annulaire (124(1)) est défini par une paroi latérale distale (103(1)) ;
**caractérisé en ce qu'**un rapport entre une longueur (D1) de la première surface étanche (123(1)) et une distance (D2) entre la paroi latérale distale (103(1)) et la poignée (178) est comprise entre 0,889 et 1,105 de sorte qu'une force de charge latérale jusqu'à 68,95 kPa (10 lbs) comme communiquée au connecteur à baïonnette mâle (102, 301), ne rompra pas le joint étanche au fluide entre la première surface étanche (123(1)) et la première surface intérieure de la structure de réception femelle (206).

2. Connecteur à baïonnette mâle (102, 301) selon la revendication 1, dans lequel la première paroi latérale distale (103(1)) définit une surface qui est orientée sensiblement perpendiculairement à un axe du premier lumen (107(1)).

3. Connecteur à baïonnette mâle (102, 301) selon la revendication 1, dans lequel la première paroi latérale distale (103(1)) définit une surface pour l'interfaçage avec une première structure à loquet (200) dans la structure de réception femelle (206) de sorte à éviter le retrait du connecteur à baïonnette mâle (102, 301) de la structure de réception femelle (206) lorsque la première structure à loquet (200) est en prise avec le premier canal annulaire (124(1)).

4. Connecteur à baïonnette mâle (102, 301) selon la revendication 1, dans lequel la poignée (178) s'étend axialement loin du premier arbre (122(1)) de sorte à définir une bride (167(1)) autour du premier arbre (122(1)) ;
et dans lequel de préférence, la bride (167(1)) définit une arête extérieure et la poignée (178) inclut une pluralité d'indentations (188) le long de l'arête extérieure de la bride (167(1)) pour faciliter la saisie de la poignée (178).

5. Connecteur à baïonnette mâle (102, 301) selon la revendication 1, dans lequel le premier canal annulaire (124(1)) définit en outre une première arête biseautée (101(1)) opposée à la première paroi latérale distale (103(1)) ;
et dans lequel de préférence une surface de la première arête biseautée (101(1)) définit un angle qui s'élève sensiblement à 45 degrés par rapport à un axe du premier lumen (107(1)).

6. Connecteur à baïonnette mâle selon la revendication 1, dans lequel la poignée (178) est positionnée pour éviter une insertion plus profonde du premier arbre (122(1)) dans la structure de réception femelle (206).

7. Connecteur à baïonnette mâle (102, 301) selon la revendication 1, comprenant en outre
un second arbre (122(2)) présentant une seconde partie d'extrémité distale et une seconde partie d'extrémité proximale et définissant en outre un second lumen (107(2)) et un second canal annulaire (124(2)) proximal et adjacent à la seconde partie d'extrémité distale, dans lequel
la seconde partie d'extrémité proximale du second arbre (122(2)) est configurée pour être en prise avec une seconde section de tubes (104(2)) et la seconde partie d'extrémité distale du second arbre (122(2)) présente une seconde surface étanche (123(2)) configurée pour être en prise avec la seconde surface intérieure d'une structure de réception femelle correspondante (206) pour créer un joint étanche au fluide ;
le second canal annulaire (124(2)) est défini par une seconde paroi latérale distale (103(2)) ; et
un rapport entre une longueur (D1) de la seconde surface étanche (123(2)) et une distance (D2) entre la seconde paroi latérale distale (103(2)) et la poignée (178) est tel qu'une force de charge latérale jusqu'à 68,95 kPa (10 lbs), comme communiquée au connecteur à baïonnette mâle (102, 301), ne rompra pas le joint étanche au fluide entre la seconde surface étanche (123(2)) et la seconde surface intérieure de la structure de réception femelle (206).

8. Connecteur à baïonnette mâle (102, 301) selon la revendication 7, dans lequel la poignée (178) relie les premier et second arbres (122(1), 122(2)) ;
dans lequel de préférence, la poignée (178) s'étend axialement loin du premier arbre (122(1)) de sorte à définir une première bride (167(1)) autour du premier arbre (122(1)) et axialement loin du second arbre (122(2)) de sorte à définir une seconde bride (167(2)) autour du second arbre (122(2)) et les première et seconde brides (167(1), 167(2)) sont reliées par une partie nervurée entre elles ; et
dans lequel en particulier la partie nervurée définit une zone évidée entre les première et seconde brides (167(1), 167(2)).

9. Connecteur à baïonnette mâle (102, 301) pour la liaison selon la revendication 1, comprenant en outre :
un second arbre (122(2)) présentant une seconde partie d'extrémité distale et une seconde partie d'extrémité proximale et définissant en outre un second lumen (107(2)), dans lequel la seconde partie d'extrémité proximale du second arbre (122(2)) est configurée pour être en prise avec une seconde section de tubes (104(2)) et la seconde partie d'extrémité distale du second arbre (122(2)) présente une seconde surface étanche (123(2)) configurée pour être en prise avec une seconde surface intérieure d'une structure de réception femelle correspondante (206) pour créer un joint étanche au fluide ; et
la poignée (178) reliant les premier et second arbres (122(1), 122(2)) ; dans lequel
la première paroi latérale distale (103(1)) est perpendiculaire par rapport à un axe du premier lumen (107(1)) et une première paroi latérale proximale présentant une arête biseautée (101(1)).

10. Connecteur à baïonnette mâle (102, 301) selon la revendication 9, dans lequel
le second arbre (122(2)) définit un second canal annulaire (124(2)) proximal et adjacent à la seconde partie d'extrémité distale,
et définit en outre une seconde tablette annulaire définissant une paroi distale du second canal annulaire (124(2)) et une seconde arête biseautée (101(2)) définissant une paroi proximale du second canal annulaire (124(2)),
la seconde tablette annulaire définit une surface qui est perpendiculaire par rapport à un axe du second lumen (107(2)) et
un rapport entre une longueur (D1) de la seconde surface étanche (123(2)) et une distance (D2) entre la seconde tablette et la poignée (178) est tel qu'une force de charge latérale jusqu'à 68,95 kPa (10 lbs), comme communiquée au connecteur à baïonnette mâle (102, 301), ne rompra pas le joint étanche au fluide entre la seconde surface étanche (123(2)) et la seconde surface intérieure de la structure de réception femelle (206).

11. Connecteur à baïonnette mâle (102, 301) selon la revendication 9, dans lequel la poignée (178) s'étend axialement loin des premier et second arbres (122(1), 122(2)) de sorte à définir une première bride (167(1)) autour du premier arbre (122(1)) et une seconde bride (167(2)) autour du second arbre (122(2)).

12. Connecteur à baïonnette mâle (102, 301) selon la revendication 10, dans lequel les premier et second arbres (122(1), 122(2)) sont reliés par une partie nervurée qui s'étend entre les première et seconde brides (167(1), 167(2)).

13. Baïonnette mâle selon la revendication 7 ou 9, dans laquelle une distance (D2) entre un premier axe central du premier lumen (107(1)) et un second axe central du second lumen (107(2)) est comprise entre 1,695 et 2,035 fois la distance (D2) entre la première paroi latérale distale (103(1)) et la poignée (178).
